# FASCICULE DE BREVET EUROPEEN

(11) **EP 3 173 460 B1**
(45) Date de publication et mention de la délivrance du brevet: **14.10.2020**
(21) Numéro de dépôt: 16306541.0
(22) Date de dépôt: 23.11.2016
(51) Int. Cl.: C10G 50/00

(54) **PROCÉDÉ D'OLIGOMÉRISATION D'OLÉFINES LÉGÈRES AU MOYEN D'UNE SECTION RÉACTIONNELLE COMPRENANT TROIS RÉACTEURS PERMUTABLES FAVORISANT LA SÉLECTIVITÉ VERS LES DISTILLATS**
OLIGOMERISATIONSVERFAHREN VON LEICHTOLEFINEN MITTELS EINES REAKTIONSABSCHNITTS, DER DREI AUSTAUSCHBARE REAKTOREN UMFASST, DIE DIE SELEKTIVITÄT GEGENÜBER DEN DESTILLATEN BEGÜNSTIGEN
METHOD FOR OLIGOMERISING LIGHT OLEFINS BY MEANS OF A REACTION SECTION COMPRISING THREE SWITCHABLE REACTORS PROMOTING THE SELECTIVITY TO DISTILLATES

(30) Priorité: 30.11.2015 FR 1561559
(43) Date de publication de la demande: 31.05.2017
(73) Titulaire: AXENS, 92508 Rueil-Malmaison Cedex (FR)
(72) Inventeur: PUCCI, Annick, 78290 Croissy sur Seine (FR); GAGNIERE, Marielle, 69100 Villeurbanne (FR)
(74) Mandataire: IFP Energies nouvelles

(56) Documents cités:
- EP-A2- 0 136 026
- FR-A1- 2 970 260
- US-A- 4 547 602
- US-A1- 2013 165 711

## Description

### DOMAINE DE L'INVENTION

L'invention concerne la production de distillat moyen par l'intermédiaire d'un procédé d'oligomérisation d'oléfines comprenant de 3 à 9 atomes de carbone. Un distillat moyen selon l'invention correspond aux fractions ayant un point d'ébullition supérieur à 140°C, du type kérosène et/ou gasoil.

### ART ANTÉRIEUR

Les procédés d'oligomérisation d'oléfines opèrent en présence de catalyseurs acides. Habituellement, l'augmentation du rendement en distillats moyens des procédés d'oligomérisation, est obtenue en opérant à plus forte sévérité. Cette plus forte sévérité est généralement obtenue au moyen d'une augmentation de la température moyenne du catalyseur et/ou au moyen d'une augmentation du temps de résidence de la charge sur ledit catalyseur par une diminution de la vitesse spatiale de la charge.

De façon conventionnelle, pour maximiser la conversion des oléfines, on installe une batterie de réacteurs à lit fixes en séries avec refroidissement intermédiaire ou refroidissement direct pour contrôler l'exothermicité de la réaction. Le premier réacteur qui reçoit la charge fraiche réactive polluée par des composés soufrés, azotés ou oxygénés est celui qui comporte le catalyseur qui se désactive en premier de par la formation de coke et l'empoisonnement en particulier par les composés azotés basiques. Le réacteur de tête se trouve donc désactivé prématurément et le changement du catalyseur est nécessaire pour maintenir la performance tandis que la température des réacteurs en aval est progressivement augmentée pour compenser leurs désactivations progressives.

Selon une autre mise en œuvre conventionnelle, des réacteurs à lit fixes, adiabatiques ou isothermes, peuvent être installés en parallèle de manière à ne traiter qu'une partie seulement du débit et à opérer de façon étagée. Dans cette configuration, il y a pratiquement toujours un réacteur qui est court-circuité pour régénérer le catalyseur ou le remplacer par du catalyseur frais. Dans cette dernière configuration, du fait du positionnement en parallèle, la charge réactive fraiche passe sur le catalyseur frais ou régénéré avec le risque d'une exothermicité incontrôlée et l'inconvénient de favoriser le cokage prématuré et le craquage.

En parallèle des réactions d'oligomérisation, il est connu de l'homme de l'art que ce type de catalyse mettant en jeu un catalyseur acide génère des réactions d'isomérisation, de dismutation de transfert d'hydrogène, de cyclisation (oléfines => paraffines + aromatiques) et de formation de coke qui sont favorisées quand on durcit la sévérité des conditions opératoires pour augmenter la conversion des oléfines. En particulier on observe la formation de paraffines légères ce qui se traduit par une baisse significative du rendement en distillat.

La demande de brevet US 2014/0135543 décrit un procédé d'oligomérisation d'oléfines permettant de maximiser le produit désiré grâce à une installation unique pouvant traiter 2 charges distinctes avec deux types de catalyseurs différents de manière indépendante.

Le brevet FR 2 873 116 décrit un procédé d'oligomérisation d'oléfines utilisant un catalyseur à base de silice-alumine. Dans ce brevet plusieurs configurations de la section réactionnelle sont décrites pour la mise en œuvre du procédé, notamment un mode de fonctionnement comprenant deux réacteurs d'oligomérisation en série avec une séparation intermédiaire des oligomères.
Les brevets EP0136026 et US4547602 décrivent un procédé d'oligomérisation.
Il existe donc un réel besoin d'optimisation des procédés d'oligomérisation d'oléfines permettant de maximiser le rendement en distillat moyen.

De manière surprenante, la demanderesse a pu démontrer que la mise en œuvre de trois réacteurs permutables au sein d'un procédé d'oligomérisation d'oléfine permettait d'obtenir de meilleurs rendements sans avoir besoin de durcir les conditions opératoires, grâce au réglage de la température de chacun des réacteurs et en particulier en imposant à chacun des réacteurs une température d'au moins 30°C inférieure à la température du réacteur précédent.

### OBJET DE L'INVENTION

L'invention concerne un procédé de production de distillat moyens comprenant au moins une étape d'oligomérisation catalytique d'oléfines comprenant de 3 à 9 atomes de carbone comme décrit par la revendication 1.

Dans ces conditions il est apparu de façon surprenante que l'installation du catalyseur le plus frais dans le dernier réacteur permet à la fois d'atteindre de hautes conversions à basse température et à la fois de limiter le craquage des fractions lourdes bouillant dans la gamme de température des distillats moyens et donc de maximiser le rendement en distillat moyens, c'est-à-dire en fractions ayant un point d'ébullition supérieur à 140°C, du type Kérosène et/ou Gasoil.

Un autre avantage de ce procédé est qu'il permet de réaliser l'oligomérisation des oléfines branchées très réactives dans le ou les premiers réacteurs en présence d'un catalyseur passivé par le dépôt de coke durant son opération dans le dernier réacteur, évitant ainsi tout risque d'emballement thermique. L'exothermicité de la réaction mettant en jeu du catalyseur frais ou régénéré est par ailleurs maitrisée en le positionnant en dernier, où il opère sur l'effluent des réacteurs localisés en amont, donc un effluent débarrassé des oléfines les plus réactives. Ainsi, au redémarrage du réacteur situé en dernière position, l'exothermicité observée habituellement est moins importante et plus aisément contrôlée, car les oléfines les plus réactives présentes dans la charge ont été consommées dans le réacteur de tête.

### DESCRIPTION DÉTAILLÉE DE L'INVENTION

L'invention concerne un procédé de production de distillats moyens par oligomérisation d'oléfines comprenant de 3 à 9 atomes de carbone et de manière préférée de 4 à 6 atomes de carbone au moyen d'une section réactionnelle comprenant trois réacteurs permutables. Le procédé selon l'invention se distingue des procédés selon l'art antérieur en ce que ledit procédé est opéré avec des réacteurs permutables comprenant chacun un catalyseur dont la fraicheur est différente, le dernier réacteur contenant le catalyseur le plus frais et opérant à une température moyenne (WABTn) inférieure à la température moyenne (WABTn-1) du réacteur le précédant directement, l'écart entre lesdites températures moyennes étant d'au moins 30°C (WABTn-1 - WABTn ≥ 30°C). Cet agencement permet d'améliorer la sélectivité vers les distillats moyens.

On entend par catalyseur frais un catalyseur n'ayant jamais été utilisé ou un catalyseur n'ayant pas été utilisé après régénération. Ainsi, un catalyseur est qualifié de plus frais lorsque son temps d'utilisation dans un réacteur, éventuellement après régénération, est plus court que les temps d'utilisation des catalyseurs présents dans les autres réacteurs.

On entend par temps d'utilisation, la durée pendant laquelle un catalyseur est mis en contact avec la charge.

On entend par réacteurs permutables des réacteurs disposés en série et utilisés de façon cyclique en répétant, après une étape a) durant laquelle la charge traverse successivement tous les réacteurs d'oligomérisation, successivement des étapes b), b'), et c) définies ci-après :
- une étape b) durant laquelle la charge est introduite dans le réacteur non désactivé situé immédiatement en aval, par rapport au sens de circulation de la charge, du réacteur désactivé, en court-circuitant le réacteur désactivé, ,
- une étape b'), simultanée à l'étape b), durant laquelle le catalyseur du réacteur désactivé est régénéré ou remplacé,
- une étape c) durant laquelle la charge traverse tous les réacteurs d'oligomérisation, le réacteur dont le catalyseur a été régénéré ou remplacé à l'étape b') étant reconnecté de manière à être situé en aval des autres réacteurs par rapport au sens de circulation de la charge.

On entend par WABTn la température moyenne dans le dernier réacteur. Les températures moyennes des autres réacteurs sont déclinées selon la terminologie suivante : WABTn-1 pour la température moyenne de l'avant dernier réacteur; WABTn-2 pour la température moyenne du réacteur placé directement en amont de l'avant dernier réacteur et ainsi de suite.

En marche stabilisée la température du dernier réacteur doit être ajustée pour finaliser la conversion et cela tant que le catalyseur en première position n'est pas passivé de sorte que la température moyenne du lit ou des lits de catalyseur en dernière position ne dépasse pas une température moyenne dite WABTn (Weighted Average Bed Temperature selon la terminologie anglo-saxonne). Conformément à l'invention la température WABTn est inférieure d'au moins moins 30°C, à la température moyenne du ou des lit s de catalyseurs dans le premier réacteur.

Le réacteur contenant le catalyseur le plus frais est installé en dernière position selon le sens de circulation de la charge. Cette méthode implique que chaque réacteur est avancé d'une place à chaque fois qu'un catalyseur est régénéré ou remplacé jusqu'à devenir le réacteur occupant la première position. Ledit premier réacteur est alors isolé pour permettre le remplacement ou la régénération du catalyseur puis est reconnecté en dernière position. Le premier réacteur contient donc toujours le catalyseur le moins frais, qui finit son cycle dans cette position jusqu'à sa passivation.

Le procédé selon l'invention comprend trois réacteurs. Les deux premiers réacteurs sont en fonctionnement pendant que le troisième réacteur n'est pas en fonctionnement. Ledit troisième réacteur est chargé et utilisé pour remplacer le second réacteur dès que nécessaire. Cette disposition permet d'assurer un fonctionnement en continu de l'unité avec deux réacteurs en fonctionnement pendant que le catalyseur du troisième réacteur est régénéré ou remplacé.

Tout type de catalyseur d'oligomérisation peut être utilisé dans le procédé selon l'invention. De manière préférée, lesdits catalyseurs sont choisis parmi les catalyseurs de type zéolitiques, de types oxydes mixtes amorphes, de types oxydes mixtes cristallisés et les résines.

Des catalyseurs zéolitiques peuvent être utilisés dans les procédés d'oligomérisation et dans ce cas, un liant peut éventuellement être ajouté, notamment pour faciliter la mise en forme de la zéolithe. En particulier, les zéolithes appartenant aux familles suivantes décrites dans l'atlas des structures zéolitiques (Atlas of Zeolite Framework Types, Ch. Baerlocher, L.B. McCusker, D.H. Oison, 6^{th} Revised Edition 2007, ELSEVIER) peuvent être utilisées : AEL, AFO, ATO, BEA, BPH, EUO, FER, FAU, IMF, ITH, MEI, MEL, MFI, MOR, MSE, MTT, MTW, MWW, OFF, SFV, SVR, TON, TUN, UZM-8. De manière plus préférée, on peut par exemple utiliser les zéolithes EU-1, ZSM-5, ZSM-11, ZSM-12, ZSM-22, ZSM-23, ZSM-35.

Les catalyseurs utilisées peuvent également être de type oxyde mixte amorphe ou non. Par exemple le brevet EP 0 463 673 décrit des catalyseurs d'oligomérisation du propylène à base de silice-alumine amorphe avec un rapport molaire silice/alumine compris entre 30/1 et 500/1 et le brevet US 4 544 791 décrit des catalyseurs d'oligomérisation d'oléfines contenant 4 atomes de carbone à base d'alumines promues par du bore ou fluorées, ou encore d'une silice-alumine amorphe ayant une teneur en silice comprise entre 60 et 95 % en masse.

Le procédé est généralement opéré à une température comprise entre 40°C et 350°C, de préférence entre 120°C et 300°C.

La pression est généralement comprise entre 0,1MPa et 15MPa, de préférence entre 2MPa et 10MPa.

La vitesse volumétrique horaire, également appelée VVH (débit de charge par volume de catalyseur ou volume de charge /(volume de catalyseur.heure)) est généralement comprise entre 0,05h⁻¹ et 5,0h⁻¹, de préférence entre 0,3h⁻¹ et 3,0h⁻¹. Ladite VVH ne représentant que la quantité de charge qui n'a pas traversé la section réactionnelle, communément appelée charge fraiche, la valeur de la VVH ne prend donc pas en compte un éventuel recyclage de charge non convertie.

### EXEMPLES

### Exemple 1 (comparatif) : 2 réacteurs en série non permutables

On utilise une charge obtenue par fractionnement de l'essence totale issue d'un craquage catalytique en lit fluidisé et contenant 56 % en poids d'oléfines. Sa teneur en soufre est de 50 ppm en poids. La charge dont la composition, déterminée par chromatographie phase gazeuse, est indiquée au tableau 1 est passée au préalable sur tamis moléculaire 13X afin d'enlever les composés azotés.

**Tableau 1: composition de la charge**

| Caractéristiques de la charge | % Poids |
|---|---|
| C4 oléfines | 1 |
| C5 oléfines | 43 |
| C6 oléfines | 12 |
| C4-C6 Paraffines | 44 |
| C5 Diènes | 800 ppm poids |
| Densité | 0,654 |

Initialement, les deux réacteurs en opération contiennent des catalyseurs frais et sont à la même WABT de 155°C. Les températures des deux réacteurs sont progressivement augmentées au cours du temps jusqu'à une température de 200°C afin de compenser la désactivation des catalyseurs. Ces augmentations de températures ont été réalisées en parallèle dans les deux réacteurs en maintenant les WABT identiques afin de maintenir une conversion constante dans le temps.

Dans cette configuration, la température WABT du second réacteur reste donc égale à la WABT du premier réacteur au cours du temps.

La charge est alors engagée dans un procédé d'oligomérisation comprenant deux réacteurs opérant dans les conditions opératoires initiales suivantes :
- Réacteur 1 : WABT1 = 200°C et VVH = 1h⁻¹, réacteur comprenant un lit de catalyseur silice-alumine extrudé de 1,6 mm de diamètre (commercialisé sous la référence IP 811 par la société Axens).
- Réacteur 2 : WABT2 = 200°C et VVH = 1h⁻¹, réacteur comprenant un lit de catalyseur identique à celui décrit pour le réacteur 1.

Le taux de conversion des oléfines comportant 5 ou 6 atomes de carbone est de 85%. Le pourcentage en poids de fraction essence (point d'ébullition inférieur à 140°C) est de 22% et le pourcentage en poids de distillat moyen (point d'ébullition supérieur à 140°C) est de 78%.

### Exemple 2 (selon l'invention) : 2 réacteurs en série, permutables

La charge est identique à celle de l'exemple 1.

Initialement, les deux réacteurs en opération contiennent le même catalyseur frais que celui de l'exemple 1 et la température WABT de chacun des réacteurs est de 155°C.

Au cours du temps on augmente indépendamment la température des deux réacteurs pour compenser la désactivation des catalyseurs. La température du réacteur en deuxième position selon le sens de circulation de la charge est maintenue plus basse que celle du réacteur en première position, tout en maintenant une conversion constante.

Lorsque le catalyseur du réacteur 2 est en milieu de cycle, le réacteur contenant celui-ci est placé en première position pour devenir le réacteur 1, un réacteur contenant du catalyseur frais est alors placé en deuxième position pour devenir le réacteur 2.

La charge est alors engagée dans un procédé d'oligomérisation opérant dans les conditions opératoires suivantes :
- Réacteur 1 : WABT1 = 190°C - VVH = 1h⁻¹, réacteur comprenant un lit de catalyseur en milieu de cycle.
- Réacteur 2 : WABT2 = 155°C - VVH = 1h⁻¹ réacteur comprenant un lit de catalyseur frais.

Le réacteur 2 opère donc à une température WABT 35°C en dessous de celle du réacteur 1.

La conversion globale des oléfines comportant 5 ou 6 atomes de carbone est de 85%. Le pourcentage en poids de fraction essence (point d'ébullition inférieur à 140°C) est de 15% et le pourcentage en poids de distillat moyen (point d'ébullition supérieur à 140°C) est de 85%.

### Exemple 3 (selon invention):

La charge est identique à celle de l'exemple 1.

Cet exemple est initialement mené dans les conditions de l'exemple 2 de manière à obtenir un réacteur 1 comportant un catalyseur proche de sa fin de cycle et un réacteur 2 comportant un catalyseur partiellement désactivé.

La charge est ensuite engagée dans un procédé d'oligomérisation opérant dans les conditions opératoires suivantes :
- Réacteur 1 : WABT1 = 250°C - VVH = 1h⁻¹, réacteur comprenant le lit de catalyseur en fin de cycle.
- Réacteur 2 : WABT2 = 185°C - VVH = 1h⁻¹, réacteur comprenant le lit de catalyseur partiellement désactivé.

La conversion globale des oléfines comportant 5 ou 6 atomes de carbone est de 85%. Le pourcentage en poids de fraction essence (point d'ébullition inférieur à 140°C) est de 17% et le pourcentage en poids de distillat moyen (point d'ébullition supérieur à 140°C) est de 83%.

A ce stade, le catalyseur du réacteur 1 opère à haute température car il est proche de la fin de son cycle, le réacteur 2 est sur le point d'être permuté en position 1, mais il est encore opéré à une température plus basse de 65°C par rapport à celle du réacteur 1 conformément à l'invention, et plus basse de 15°C par rapport à celle du réacteur 2 de l'exemple 1 (comparatif).

**Tableau 2 : comparaison des résultats obtenus dans les exemples 1, 2 et 3**

| Sélectivités | Exemple 1 (Comparatif) | Exemple 2 (Invention) | Exemple 3 (Invention) |
|---|---|---|---|
| % poids fraction essence (point d'ébullition inférieur à 140°C) | 22 | 15 | 17 |
| % poids distillat moyen (point d'ébullition supérieur à 140°C) | 78 | 85 | 83 |
| % conversion des oléfines comportant 5 ou 6 atomes de carbone | 85 | 85 | 85 |

Le procédé selon l'invention (Exemples 2 et 3) permet de maximiser la sélectivité de la réaction d'oligomérisation vers les distillats par rapport aux procédés de l'art antérieur (Exemple 1) en minimisant le recraquage en essence des oligomérats lourds formés par la réaction d'oligomérisation.

## Revendications

1. Procédé de production de distillat moyens comprenant au moins une étape d'oligomérisation catalytique d'une charge comportant des oléfines comprenant de 3 à 9 atomes de carbone dans lequel la section réactionnelle comporte trois réacteurs positionnés en série et permutables contenant chacun au moins un catalyseur des réactions d'oligomérisation, lesdits catalyseurs étant identiques ou différents, et dans lequel le réacteur le plus en aval selon le sens de circulation de ladite charge contient le catalyseur dont le temps d'utilisation est plus court que le temps d'utilisation des catalyseurs présents dans les autres réacteurs et opère à une température moyenne (WABTn) inférieure à la température moyenne (WABTn-1) du réacteur le précédant directement, l'écart entre lesdites températures moyennes étant d'au moins 30°C (WABTn-1 - WABTn ≥ 30°C),
dans lequel les deux premiers réacteurs sont en fonctionnement pendant que le troisième réacteur n'est pas en fonctionnement de manière à assurer un fonctionnement en continu de l'unité avec deux réacteurs en fonctionnement pendant que le catalyseur du troisième réacteur est régénéré ou remplacé,
dans lequel les réacteurs positionnés en série et permutables sont utilisés de façon cyclique en répétant, après une étape a) durant laquelle la charge traverse successivement tous les réacteurs d'oligomérisation, successivement des étapes b), b'), et c) définies ci-après :
• une étape b) durant laquelle la charge est introduite dans le réacteur non désactivé situé immédiatement en aval, par rapport au sens de circulation de la charge, du réacteur désactivé, en court-circuitant le réacteur désactivé,
• une étape b'), simultanée à l'étape b), durant laquelle le catalyseur du réacteur désactivé est régénéré ou remplacé,
• une étape c) durant laquelle la charge traverse tous les réacteurs d'oligomérisation, le réacteur dont le catalyseur a été régénéré ou remplacé à l'étape b') étant reconnecté de manière à être situé en aval des autres réacteurs par rapport au sens de circulation de la charge
et dans lequel, chaque réacteur est avancé d'une place à chaque fois qu'un catalyseur est régénéré ou remplacé jusqu'à devenir le réacteur occupant la première position, ledit premier réacteur est alors isolé pour permettre le remplacement ou la régénération du catalyseur puis est reconnecté en dernière position.

2. Procédé selon la revendication 1, dans lequel lesdits réacteurs contiennent un lit de catalyseur.

3. Procédé selon l'une quelconque des revendications 1 à 2, dans lequel lesdits réacteurs contiennent plusieurs lits de catalyseur.

4. Procédé selon l'une quelconque des revendications précédentes, dans lequel lesdits catalyseurs sont choisis parmi les catalyseurs de type zéolitiques, de types oxydes mixtes amorphes, de types oxydes mixtes cristallisés et les résines.

5. Procédé selon l'une quelconque des revendications précédentes, dans lequel la température est comprise entre 40°C et 350°C.

6. Procédé selon l'une quelconque des revendications précédentes, dans lequel la pression est comprise entre 0,1 MPa et 15 MPa.

7. Procédé selon l'une quelconque des revendications précédentes, dans lequel la vitesse volumétrique horaire est comprise entre 0,05h⁻¹ et 5,00h⁻¹.

## Patentansprüche

1. Verfahren zur Herstellung von Mitteldestillat, umfassend mindestens einen Schritt der katalytischen Oligomerisation eines Einsatzstoffs, der Olefine mit 3 bis 9 Kohlenstoffatomen umfasst, wobei der Reaktionsteil drei in Reihe angeordnete und permutable Reaktoren umfasst, die jeweils mindestens einen Katalysator für Oligomerisationsreaktionen enthalten, wobei die Katalysatoren gleich oder verschieden sind, und wobei der in Richtung der Strömung des Einsatzstoffs am weitesten stromabwärts angeordnete Reaktor den Katalysator, dessen Verwendungszeit kürzer ist als die Verwendungszeit der in den anderen Reaktoren vorliegenden Katalysatoren, enthält und bei einer mittleren Temperatur (WABTn) betrieben wird, die unter der mittleren Temperatur (WABTn-1) des unmittelbar vorhergehenden Reaktors liegt, wobei die Differenz zwischen den mittleren Temperaturen mindestens 30 °C beträgt (WABTn-1 - WABTn ≥ 30 °C),
wobei die beiden ersten Reaktoren in Betrieb sind, während der dritte Reaktor nicht in Betrieb ist, um einen kontinuierlichen Betrieb der Einheit mit zwei Reaktoren im Betrieb zu gewährleisten, während der Katalysator des dritten Reaktors regeneriert oder ersetzt wird,
wobei die in Reihe geschalteten und permutablen Reaktoren in zyklischer Weise verwendet werden, indem nach einem Schritt a), bei dem der Einsatzstoff nacheinander alle der Oligomerisationsreaktoren nacheinander durchläuft, die nachstehend definierten Schritte b, b') und c) nacheinander wiederholt werden:
• ein Schritt b), bei dem der Einsatzstoff in den nicht desaktivierten Reaktor, der bezogen auf die Richtung der Strömung des Einsatzstoffs unmittelbar stromabwärts des desaktivierten Reaktors angeordnet ist, eingetragen wird, wobei der desaktivierte Reaktor übergangen wird,
• einen Schritt b'), der gleichzeitig mit Schritt b) erfolgt, bei dem der Katalysator des desaktivierten Reaktors regeneriert oder ersetzt wird,
• einen Schritt c), bei dem der Einsatzstoff alle Oligomerisationsreaktoren durchläuft, wobei der Reaktor, dessen Katalysator in Schritt b') regeneriert oder ersetzt wurde, so wieder angeschlossen wird, dass er bezogen auf die Richtung der Strömung des Einsatzstoffs stromabwärts angeordnet ist,
und wobei jeder Reaktor jedes Mal, wenn ein Katalysator regeneriert oder ersetzt wird, eine Stelle vorgerückt wird, bis er zu dem die erste Position einnehmenden Reaktor wird, und der erste Reaktor dann isoliert wird, um den Ersatz oder die Regeneration des Katalysators zu ermöglichen, und dann in der letzten Position wieder angeschlossen wird.

2. Verfahren nach Anspruch 1, wobei die Reaktoren eine Katalysatorschüttung enthalten.

3. Verfahren nach einem der Ansprüche 1 bis 2, wobei die Reaktoren mehrere Katalysatorschüttungen enthalten.

4. Verfahren nach einem der vorhergehenden Ansprüche, wobei die Katalysatoren aus Katalysatoren vom Zeolith-Typ, Katalysatoren vom Typ amorphe gemischte Oxide, Katalysatoren vom Typ kristalline gemischte Oxide und Harzen ausgewählt sind.

5. Verfahren nach einem der vorhergehenden Ansprüche, wobei die Temperatur zwischen 40 °C und 350 °C liegt.

6. Verfahren nach einem der vorhergehenden Ansprüche, wobei der Druck zwischen 0,1 MPa und 15 MPa liegt.

7. Verfahren nach einem der vorhergehenden Ansprüche, wobei die Katalysatorbelastung zwischen 0,05 h⁻¹ und 5, 00 h⁻¹ liegt.

## Claims

1. Process for production of middle distillates, comprising at least one step of catalytic oligomerization of a feed comprising olefins comprising from 3 to 9 carbon atoms, in which the reaction section comprises three reactors which are positioned in series and which are switchable, each containing at least one catalyst for oligomerization reactions, said catalysts being identical or different, and in which the reactor which is the furthest downstream in the direction of circulation of said feed contains the catalyst with a period of service which is shorter than the period of service of the catalysts present in the other reactors and is operated at an average temperature (WABTn) which is lower than the average temperature (WABTn-1) of the reactor directly preceding it, the difference between said average temperatures being at least 30°C (WABTn-1 - WABTn ≥ 30°C),
in which the first two reactors are in operation while the third reactor is not in operation, to ensure continuous operation of the unit with two reactors in operation while the catalyst of the third reactor is regenerated or replaced,
in which the reactors which are positioned in series and which are switchable are used in a cyclic manner by repeating, after a step a) during which the feed passes in succession through all of the oligomerization reactors, steps b), b') and c) defined below in succession:
• a step b) during which the feed is introduced into the non-deactivated reactor located immediately downstream, with respect to the direction of circulation of the feed, of the deactivated reactor, short-circuiting the deactivated reactor,
• a step b'), which is simultaneous with step b), during which the catalyst of the deactivated reactor is regenerated or replaced,
• a step c) during which the feed passes through all of the oligomerization reactors, the reactor with the catalyst which has been regenerated or replaced in step b') being reconnected in a manner such as to be situated downstream of the other reactors with respect to the direction of circulation of the feed
and in which each reactor is moved up one place each time a catalyst is regenerated or replaced, until it becomes the reactor occupying the first position, and said first reactor is then isolated to allow the replacement or regeneration of the catalyst and then is reconnected in last position.

2. Process according to Claim 1, in which said reactors contain one bed of catalyst.

3. Process according to either one of Claims 1 and 2, in which said reactors contain a plurality of beds of catalyst.

4. Process according to any one of the preceding claims, in which said catalysts are selected from zeolitic type catalysts, mixed amorphous oxide type catalysts, mixed crystalline oxide type catalysts and resins.

5. Process according to any one of the preceding claims, in which the temperature is in the range 40°C to 350°C.

6. Process according to any one of the preceding claims, in which the pressure is in the range 0.1 MPa to 15 MPa.

7. Process according to any one of the preceding claims, in which the hourly space velocity is in the range 0.05 h⁻¹ to 5.00 h⁻¹.
